# EUROPEAN PATENT SPECIFICATION

(11) **EP 3 878 441 B1**
(45) Date of publication and mention of the grant of the patent: **22.05.2024**
(21) Application number: 21161279.1
(22) Date of filing: 08.03.2021
(51) Int. Cl.: A61K 31/122, A61K 31/197, A61K 31/198, A61P 35/00, A61P 37/00

(54) **ALOE EMODIN AND ESTER DERIVATIVES THEREOF FOR TREATING SSTR2 AND/OR SSTR5 OVEREXPRESSING CANCERS**
VERWENDUNG VON ALOE EMODIN UND DESSEN ESTERDERIVATEN ZUR BEHANDLUNG VON SSTR2- UND/ODER SSTR5-ÜBEREXPRESSIERENDEN TUMOREN
UTILISATIONS DE L'ALOE EMODINE ET DE SES DÉRIVÉS D'ESTER POUR LE TRAITEMENT DES TUMEURS CARACTERISÉES PAR UNE SUREXPRESSION DE SSTR2 ET/OU SSTR5

(30) Priority: 09.03.2020 IT 202000004939
(43) Date of publication of application: 15.09.2021
(73) Proprietor: Pecere, Teresa, 35143 Padova (IT)
(72) Inventor: PECERE, Teresa, 35143 PADOVA (IT); Giorgio, PALU', 35036 Montegrotto (IT); Modesto, CARLI, deceased (IT)
(74) Representative: Zaccaro, Elisabetta

(56) References cited:
- WO-A1-02/090313
- WO-A2-2011/089602
- US-A1- 2018 200 217
- "Abstracts for the International Investigative Dermatology 2008", JOURNAL OF DERMATOLOGICAL SCIENCE, ELSEVIER, AMSTERDAM, NL, vol. 50, no. 2, 1 April 2008 (2008-04-01), pages e1-e285, XP022575973, ISSN: 0923-1811
- HU BOYANG ET AL: "Aloe-emodin from rhubarb (Rheum rhabarbarum) inhibits lipopolysaccharide-induced inflammatory responses in RAW264.7 macrophages", JOURNAL OF ETHNOPHARMACOLOGY, ELSEVIER IRELAND LTD, IE, vol. 153, no. 3, 29 March 2014 (2014-03-29), pages 846-853, XP028655249, ISSN: 0378-8741, DOI: 10.1016/J.JEP.2014.03.059

## Description

### FIELD OF THE INVENTION

The present invention relates to 1,8-dihydroxy-3-[hydroxymethyl]-anthraquinone and its derivatives and their use as drugs in the treatment of diseases in which somatostatin receptor 2 (SSTR2) and/or somatostatin receptor 5 (SSTR5) are overexpressed.

### STATE OF THE ART

In the era of combinatorial chemistry and high-throughput screening of large compound libraries development of natural products has been neglected. However, despite steady progress in the above fields, treatment modalities of a number of pathologies are unsatisfactory and there is still an unmet need for new drug discovery.

Somatostatins (SSTs) form a family of cyclopeptides that are mainly produced by normal endocrine, gastrointestinal, immune and neuronal cells, as well as by certain tumours. The highly potent somatostatin peptides SST-1 4 and SST-28 contain 14 and 28 amino acids, respectively.

SSTs are unique in their broad inhibitory effects on both endocrine secretion - for example, of growth hormone, insulin, glucagon, gastrin, cholecystokinin, vasoactive intestinal peptide and secretin- and exocrine secretion - for example, of gastric acid, intestinal fluid and pancreatic enzymes. This pan-antisecretory profile has led to exploratory clinical trials of natural SST-14 for the treatment of a range of conditions, including: diabetes type I and II; neuroendocrine and hypersecretory tumours; neuronal and gastrointestinal disorders, including bleeding gastric ulcers, pancreatitis, complications due to pancreatic surgery and pancreatic fistulae.

Somatostatins are also known to have modulatory effects on immune response and their anti-proliferative, anti-angiogenic, and analgesic properties make them attractive candidates for a therapeutic use in immune-mediated diseases, such as rheumatoid arthritis. It would therefore seem important to decrease the levels of inflammatory mediators in macrophages standing the role that inflammation plays in autoimmune or autoinflammatory disorders, neurodegenerative disease, or cancer. However, obstacles exist to the development of new more effective and less toxic drugs that can also treat the signs and symptoms of acute inflammation and the long-term consequences of chronic inflammatory diseases.

Although a number of studies indicated that SST-14 is efficacious under certain conditions, they also showed that its full therapeutic potential cannot be exploited owing to its short plasma half-life of <3 min.

Metabolically stable compounds with SST-like properties were expected to show an improved clinical profile. So current SST analogues are either peptidic or non-peptidic compounds, including selective agonists for sstrs, as well as compounds with universal binding profiles mimicking natural SST.

Therapeutic agents currently used for a number of the previously listed pathologies are not entirely effective, are rather nonspecific, and have multiple adverse side effects.

The need and importance is increasingly felt for the identification of alternative compounds which act specifically on the somatostatin receptors, and that don't have the drawback cited above.

It is therefore object of the present invention the development of a novel use of known compounds, which surprisingly show enhanced activity in the treatment of diseases in which the somatostatin 2 and somatostatin 5 receptors are overexpressed.

### SUMMARY OF THE INVENTION

The present invention concerns the use of 1,8-dihydroxy-3-[hydroxymethyl]-anthraquinone or derivatives thereof, in the treatment of diseases in which somatostatin receptor 2 (SSTR2) and/or somatostatin receptor 5 (SSTR5) are overexpressed, wherein said derivatives are 3'-esters of 1,8-dihydroxy-3-[hydroxymethyl]-anthraquinone with amino acids, as defined in claim 1.

The problem underlying the present invention is that of making available compounds which are effective in the treatment of pathologies chosen from the group consisting of neuroblastoma, neuroendocrine tumors or primitive neuroectodermal tumors (pNETS)in which the overexpression of the somatostatin receptors 2 and/or 5 is implied, in order to permit the manufacture of medicaments destined for the therapy of the pathologies.

### BRIEF DESCRIPTION OF THE DRAWINGS

The characteristics and advantages of the present invention will be apparent from the detailed description reported below, from the Examples given for illustrative and non-limiting purposes, and from the annexed Figures 1-4:
Figure 1A - Cell viability assay. Cells were treated with different concentrations of AE for 72 h. The viability was compared to the untreated cells. The plate absorbance (ABS) was measured in a spectrophotometer, at 620 nm wavelength.
Cell survival was expressed as a percentage calculated as follows [(ABS AE treated cells - ABS Background)/(ABS Vehicle treated cells - ABS Background)] x100 with MTT analysis.
Figure 1B - Sstr2 and sstr5 mRNA gene expression. IMR5 and MRC5 cells were exposed to AE (50µM) and SST14 (10µM) for different time points (5,30,60,120 min). Sstr5 gene was not expressed in MRC5 cells.
Figure 1C - Western-blot analyses: SSTR5 and SSTR2 protein expression after AE treatment (50µM) in AE-sensitive cells (IMR5) and in AE non-sensitive cells (MRC5). Densitometric analysis of data from representative of three experiments was presented as fold increase relative control.
Figure 2. Molecular docking predicted binding modes for Aloe-emodin in complex with the SSTR2 (Figure 2A) and the SSTR5 homology model (Figure 2B); residues involved in molecular recognition are labeled while hydrogen bond between ligands and the receptor are indicated with an arrow.
Figure 3A - Cell viability modulation. IMR5 cells proliferation was tested in the presence of SSTR2 or SSTR5 Abs and SST14. After 24 hours of co-exposition to AE (50 µM) and to natural ligand or Abs the cells were evaluated for viability. The plate absorbance (ABS) was measured in a spectrophotometer, at 620 nm wavelength. Cell survival was expressed as a percentage and calculated as follows: [(ABS AE treated cells - ABS Background)/(ABS Vehicle treated cells - ABS Background)] x100.
Figure 3B - Confocal microscopy analyses. IMR5 and MRC5 cells were pre-treated for 20 min with SSTR2, SSTR5 Abs or SST14 and relative fluorescence amount in the cells was evaluated in 3 min from AE administration. AE fluorescence (F) was expressed as a percentage collected during the incorporation and was evaluated (ΔF%) as medium of 30 regions of interest (ROIs): [(maximum value of fluorescence - basal fluorescence)/ basal fluorescence]x100.
Figure 3C - SiRNA knockdown. IMR5 cells were transfected with siRNA for somatostatin receptor 5. Western blot analysis for SSTR5 protein expression shown that gene silencing occurred 48 h after siRNA transfection and was stable for 72 h. IMR5 transfected cells were treated with AE (50 µM) for 24 h and evaluated for the proliferation inhibition.
Figure 4A - Immunohistochemistry for SSTR2. Representative immunohistochemistry for SSTR2 in archival neuroblastoma surgical specimens showing strong immunoreactivity immunoreactivity. Scale bar, 100 µm.
Figure 4B - SSTR2 H-score in neuroblastoma surgical specimens.
Figure 4C - Immunohistochemistry for SSTR5. Representative immunohistochemistry for SSTR5 in archival neuroblastoma surgical specimens showing faint (case 1) or absent (case 2 and 3). Scale bar, 100 µm.
Figure 4D - SSTR5 H-score in neuroblastoma surgical specimens.
Figure 5 - Activity of AE on cytokine IL-1 β expression. Activated macrophages were incubated with AE (0.1-50 µM) or with vehicle only. After 60 minutes LPS (100 ng/ml) or vehicle only (ns) was added to the medium. The cells were incubated for 24 hours. Culture medium was collected for quantification of the cytokine IL-1β.
Figure 6 - Activity of AE on caspase 1 activation. AE-treated macrophages were then incubated with a fluorescent probe capable of irreversibly binding the active Caspase-1 and then harvested and labeled with an anti-CD11b fluorescent antibody. The FACS analysis evaluates the percentage of macrophages with double positivity (CD11b + caspase1 activated).

### DETAILED DESCRIPTION OF THE INVENTION

The present invention concerns the use of 1,8-dihydroxy-3-[hydroxymethyl]-anthraquinone or derivatives thereof, in the treatment of diseases in which somatostatin receptor 2 (SSTR2) and/or somatostatin receptor 5 (SSTR5) are overexpressed, wherein said derivatives are 3-esters of 1,8-dihydroxy-3-[hydroxymethyl]-anthraquinone with amino acids chosen from the group consisting of lysine, alanine, tryptophan, isoleucine, tyrosine and GABA, wherein said diseases are tumors chosen from the group consisting of neuroblastoma, neuroendocrine tumors or primitive neuroectodermal tumors (pNETS).

By the term "Aloe-emodin" or "AE" as used herein, it is meant to comprise the natural anthraquinone 1,8-dihydroxy-3-[hydroxymethyl]-anthraquinone, produced by different species of well-known plants, such as Aloe and Rheum, as well as the annelid worms of the Tomopteris genus.

By "AE-derivatives" or "derivatives" we intend to comprise ester derivatives of 1,8-dihydroxy-3-[hydroxymethyl]-anthraquinone, wherein the ester is and amino acid ester such as the lysin ester of AE: AE-lysine. Other derivates are aloe-emodin derivatives in position 3' (bearing either a positive or negative charge) exhibiting improved solubility properties. The aloe-emodin derivatives of formula (I) 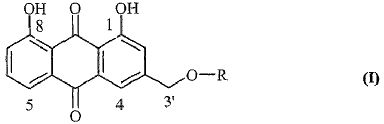 where R can be the radical of: a saturated or unsaturated C2-C6 linear or branched chain aliphatic polycarboxylic acid, or an arylic polycarboxylic acid or an amino acid or an acetal with an amino sugar or a group of an inorganic acid, as described in WO02/090313.

In the aloe-emodin derivatives of formula (I) radical R on the hydroxyl group in position 3' can be: a saturated or unsaturated aliphatic polycarboxylic acid with a linear or branched chain of from 2 to 6 carbon atoms, also substituted on the aliphatic chain by suitable hydrophilic groups. When R is an aliphatic acid of group a), said acid is preferably selected from one of the following groups: (i) linear saturated aliphatic bicarboxylic acid, e. g. oxalic, malic, succinic, glutaric, adipic, pimelic, diglycolic acids; (ii) or a branched one; (iii) or an unsaturated one, e. g. maleic acid; (iv) or a tricarboxylic acid, e. g. citric acid; an arylic polycarboxylic acid also substituted on the aromatic ring by small hydrophilic residues, such as for example OH, GH₂0H and equivalents. When R is an aromatic acid of group b) said acid is preferably selected from the group consisting of aromatic systems containing one or more rings, hydrophilic substituents, if any, and at least two carboxylic groups, such as for example phthalic acid and 1,2,4, benzenetricarboxylic acid; an amino acid (amino group in α-or in other positions), e. g. lysine, alanine, isoleucine, tyrosine, tryptophan and GABA; an acetal with an amino sugar, such as daunosamine and equivalents; the residue of an inorganic acid, such as for example phosphoric acid and equivalents.

Furthermore, in the derivatives forming the object of the present invention, in which R belongs to groups a) and b), the non-esterified carboxylic groups can be in the free or salified form; in the latter case, pharmaceutical acceptable and known counterions may be used, sodium or potassium being especially preferred.

In the derivatives forming the object of the present invention, in which R belongs to groups c) and d), the amine group can be in the free or salifie form; in the latter case, pharmaceutically acceptable and known anionic groups may be used, acetate, trifluoroacetate, nitrate, chloride, bromide, acid sulphate being especially preferred.

The aloe-emodin derivatives according to the claimed invention are: AE-lysine, AE-alanine, AE-isoleucine, AE-tyrosine, AE-tryptophan and AE-GABA.

As will be evident in the Examples section, results of gene expression, western blotting, and imaging techniques, indicate that SSTRs were expressed in a different way in neuroectodermal cells and in AE-sensitive and non-sensitive cells and that AE has the biological and molecular characteristic to be recognized by SSTR2 and SSTR5. In particular, these receptors are induced after AE exposition. Moreover, a different pattern of SSTR2 and SSTR5 expression is shown in AE-sensitive and non- sensitive cells, that correlates with the differential cytotoxic potential of AE, receptor knockdown and competition experiment. These data are corroborated by preliminary molecular modeling studies showing how AE can be recognized by SSTR2 and SSTR5 receptors establishing, in the orthosteric binding sites, a network of stabilizing interactions.

In a further aspect, the invention provides the use of 1,8-dihydroxy-3-[hydroxymethyl]-anthraquinone or derivatives thereof, wherein the amino acids of said 3'-esters of 1,8-dihydroxy-3-[hydroxymethyl]-anthraquinone with amino acids are chosen from the group consisting of lysine, alanine, isoleucine, tyrosine, tryptophan and GABA.

Still more preferred, is the aloe-emodin derivative AE-lysine and AE-Lysine(CF₃COO)₂ of formula:

AE-Lys(CF₃COO)₂

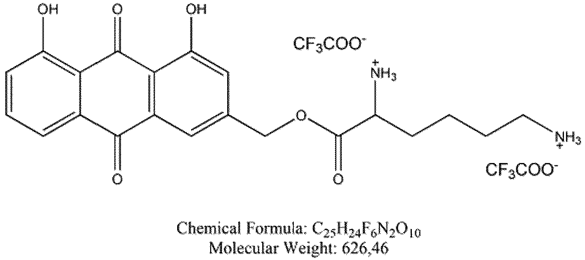

As described in claim 1, the invention provides the use of 1,8-dihydroxy-3-[hydroxymethyl]-anthraquinone or derivatives thereof in the treatment of diseases in which somatostatin receptor 2 (SSTR2) and/or somatostatin receptor 5 (SSTR5) are overexpressed, wherein said tumors are neuroblastoma, neuroendocrine tumors or primitive neuro ectodermal tumors (PNETs).

Without being bound to any theory, the data herein provided is the molecular and biological explanation of the AE selectivity.

Moreover, the observations on the receptor-mediated mode of action of the anthraquinone compound, combined with the strong and widespread expression of SSTR2 in human neuroblastoma specimens, prompt to a further development of AE as a personalized antitumor agent and to explore its potential use for other biological application mediated by somatostatin receptors.

In a further aspect, the invention provides the use of 1,8-dihydroxy-3-[hydroxymethyl]-anthraquinone or derivatives thereof, wherein said 1,8-dihydroxy-3-[hydroxymethyl]-anthraquinone is in the form of pharmaceutical formulations commonly used for drug administration by the parenteral and oral routes as well as of formulations for local administration.

In a further aspect, the invention provides the use of 1,8-dihydroxy-3-[hydroxymethyl]-anthraquinone or derivatives thereof, wherein said 1,8-dihydroxy-3-[hydroxymethyl]-anthraquinone is in the range 5 to 500 mg for unit administration.

### EXAMPLES

Reference is now made to the following examples, which together with the above descriptions illustrate some embodiments of the invention.

### Example 1

### COMPOUNDS

Aloe-Emodin (AE) was provided by Angelini S.p.A. and it was dissolved in DMSO stock solution of 200mM.

Somatostatin was purchased from Sigma-Aldrich (Sigma-Aldrich, Milan, Italy). All compounds were stored at -20°C.

### CELL CULTURE

Neuroblastoma cells (IMR5), human lung fibroblasts (MRC5), cervix epithelioid carcinoma cells (HeLa) and hepatocellular carcinoma cells (Huh7) were purchased from ATCC (American Type Culture Collection, Manassas, VA), human foreskin fibroblasts (HuF) were derived from primary tissue in our laboratory, melanoma cells (M19-Mel) and small cell lung carcinoma (DMS114) were from the National Cancer Institute (NCI, New York). MRC5, M19Mel, HeLa, HuF, and Huh7 cells were grown in DMEM medium, DMS114 and IMR5, were grown in RPMI-1640 medium (Lifetechnologies Gibco, Milan, Italy). Culture medium was supplemented with 10% heat-inactivated fetal bovine serum (Lifetechnologies Gibco, Milan, Italy), 100 units/ml penicillin and 100µg/ml streptomycin (Lifetechnologies Gibco, Milan, Italy). All cell lines were grown at 37°C with 5% CO₂ humidified atmosphere.

### CELL VIABILITY ASSAY

Cells were seeded in 96-well plates in regular growth medium. After 24 h of incubation, medium was discarded and replaced by 100 µL of regular growth medium supplemented with different concentrations of AE for 72 h. Then, cells were washed twice with PBS, and cell viability was evaluated using Cell Proliferation Kit I (Cell Proliferation Kit I, Roche Diagnostics GmbH, Mannheim, Germany) according to the manufacturer's instructions. All assays included cell treatment with the vehicle (DMSO) dissolved in growth medium at 0,05% concentration. All experiments were conducted at least in triplicate.

### RESULTS OF AE CYTOTOXICITY

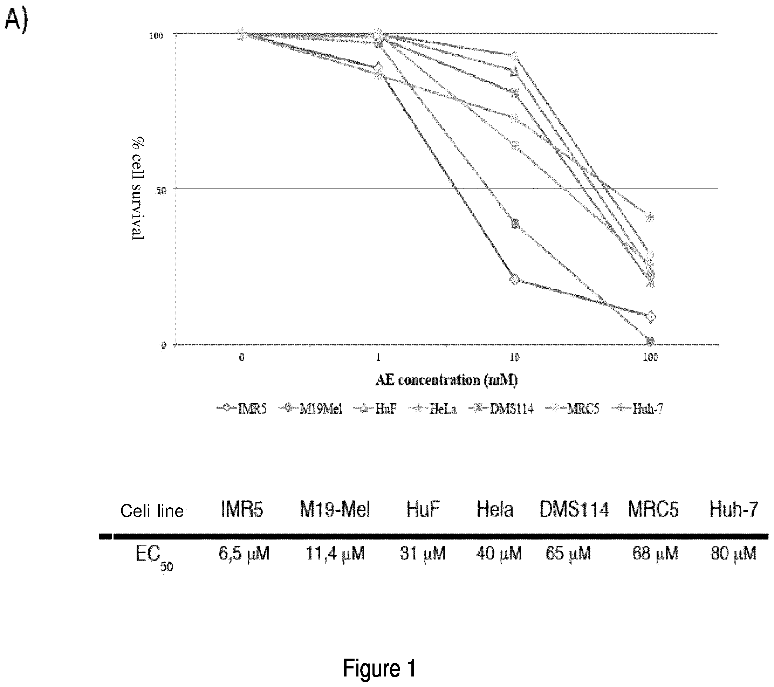

When AE cytotoxicity was evaluated on a panel of cell lines of different embryonic origin, a more significant cytotoxic activity was observed on IMR5 and M19-Mel cells vis a vis HuF, HeLa, DMS114, MRC5 and Huh-7 cells. Noteworthy M19-Mel, IMR5, and DMS114 cells are of neuroectodermal origin, whereas HuF, HeLa, MRC5 and Huh cells are of mesodermal and ectodermal origin (Fig.1A).

### Example 2

### ANALYSES OF RNA EXPRESSION

Real-time PCR analyses were carried out to quantify expression of the selected sstrs genes in IMR5, DMS114, and MRC5 cells. Total RNA was isolated using the Qiagen RNeasy Mini Kit in accordance with the manufacturer's instructions. cDNA synthesis were performed using GeneAmp^{®} RNA PCR Core Kit (Roche, New Jersey, U.S.A.). The expression levels of sstrs mRNA were evaluated by qualitative RT-PCR using the specific primers. Of the two cell lines of neuroectodermal origin, IMR5 expressed SSTR2 and 5, whereas DMS114 expressed no somatostatin receptor. MRC5, taken as a cell line representative of non-neuroectodermal origin, expressed mRNA for SSTR1 and 2.

The primers used in these experiments are:

**Table 1**

| Primers | Sequence | SEQ ID NO: |
|---|---|---|
| β-actin | 5'-ATGTCAAACGTGCGAGTGTC-3' | SEQ ID NO:1 |
| | 5'-TCTCTGCAGTGCTTCTCCAA-3' | SEQ ID NO:2 |
| SSTR1 | 5'- GCCTTATCTTTCCCGAGTGAACACC-3' | SEQ ID NO:3 |
| | 5'- GGACCTGAGTGAACCATTCGCAAA -3' | SEQ ID NO:4 |
| SSTR2 | 5'- ACTCCCTGCACCATCATCGTT-3' | SEQ ID NO:5 |
| | 5'- TGGAATTTCATGTCTGCATTGCGTA -3' | SEQ ID NO:6 |
| SSTR3 | 5'- GGCGTTAGTCTCAGAATCTCGTG -3' | SEQ ID NO:7 |
| | 5'- TGCACCTCAGTTTCGTCATATGGA -3' | SEQ ID NO:8 |
| SSTR4 | 5'- CATGGTCGCTATCCAGTGCA-3' | SEQ ID NO:9 |
| | 5'- GTGAGACAGAAGACGCTGGTGAACAT -3' | SEQ ID NO:10 |
| SSTR5 | 5'- TCTGCTAAGTTAAAGACACCCGAAA -3' | SEQ ID NO:11 |
| | 5'- CAGCCACACTCCCCTTACACC -3' | SEQ ID NO:12 |

Relative quantitative PCR (qPCR) was carried out in 25 µl final volume utilizing 3 µl of cDNA, 0.2 µM of each specific primers, using SYBRgreen reagents according to the manufacturer's instructions. PCR reactions comprised 40 cycles after an initial denaturation step (95°C, 10 min) according to the parameters: denaturation at 95°C, 15 sec and annealing/extension at 60°C, 1 min, in an ABI PRISM 7700 Sequence Detection system. To normalize the RNA amount of the extracted samples, real-time PCR analysis of the human GAPDH cDNA was carried out. Moreover, we optimized the method for relative quantification with sequence-specific DNA probes for sstr2 and sstr5 genes TaqMan reagents using kit TaqMan Gene Expression Assays according to the manufacturer's instructions (Applied Biosystem, Milan, Italy). The relative quantification of a target template in the samples was evaluated using the 2^{-ΔΔCT} method. All experiments were conducted in triplicate. The primers used in these experiments are:

**Table 2**

| Primers | Sequence | SEQ ID NO: |
|---|---|---|
| GAPDH | 5'-CCACTCCTCCACCTTTGACG-3' | SEQ ID NO:13 |
| | 5'-CATGAGGTCCACCACCCTGT-3' | SEQ ID NO:14 |
| SSTR2 | 5'-TATGTCATCCTCCGCTATGCC-3' | SEQ ID NO:15 |
| | 5'-CAGAGCCACCTGCATAGCC-3' | SEQ ID NO:16 |
| SSTR5 | 5'-CCCTTCTTCACCGTCAACAT-3' | SEQ ID NO:17 |
| | 5'-CTTGCGGAGGCACAGAACCTT-3' | SEQ ID NO:18 |

### RESULTS OF ANALYSES OF RNA EXPRESSION

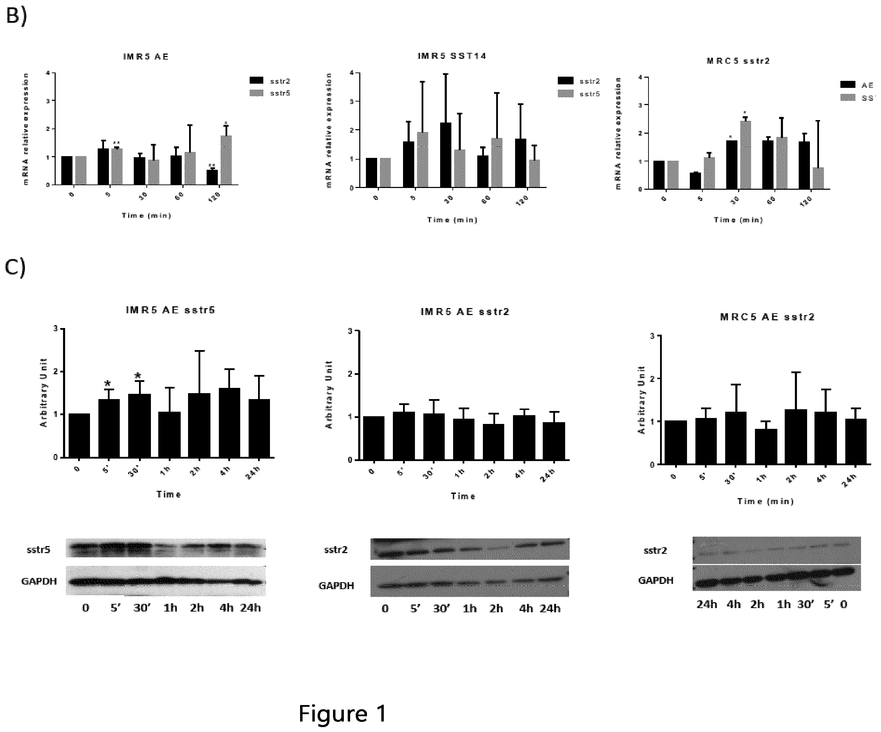

Sstr5 gene expression was significantly up-regulated in IMR5 cells by exposition to AE. At variance, sstr2 and sstr5 genes were both up-regulated by somatostatin-14 in this cell line (fig.1B). Sstr5 gene was not expressed in MRC5 cells but sstr2 gene was significantly upregulated both by AE and SST14 (fig.1B).

### WESTERN-BLOT ANALYSES

IMR5 and MRC5 cells were seeded and incubated with or without AE (50 µM). The collected cells were solubilized on ice in RIPA lysis buffer (SIGMA, Milan, Italy) supplemented with complete protease inhibitor cocktail tablet (Promega, Milan, Italy). Followed by centrifugation at 14,000 rpm for 5 min at 4°C. The protein concentration was determined by BCA assay (Pierce, Thermo Fisher), using BSA as a standard according to the manufacturer's instructions. Equal amount of total proteins (40µg) were subject to Western blot analyses. The membranes were blocked with 5% w/v nonfat dry milk in PBS with 0.1% Tween-20 and blots were probed with specific antibodies (Ab) to SSTR2, SSTR5 and GAPDH (SIGMA, Milan, Italy). Blots were visualized with a peroxidase-conjugated anti-rabbit IgG secondary antibody (Santa Cruz, CA, USA) and developed with enhanced chemiluminescence reagents (Pierce Thermo Fisher, Milan, Italy). Pixels intensity of the visualized bands was determined by using Image J software (W. S. Rasband, NIH, Bethesda, MD [http://rsb.info.nih.gov/ij/]). All experiments were conducted in triplicate.

### RESULTS OF WESTERN-BLOT ANALYSIS

Western blot analyses confirmed the above findings of receptor up-regulation by AE exposition in IMR5 cells, with the exception of SSTR2 protein whose level did not significantly increase both in IMR5 and MRC5 cells (Fig. 1C).

### Example 3

### MOLECULAR MODELING

In order to find a plausible role for these receptors in AE-sensitive cells, molecular docking simulations were exploited using the two SSTR subtypes, that were significantly expressed and upregulated.

Since no crystallographic structure of the somatostatin receptor is yet available, two homology models were constructed, using as template the recently published crystal structure of the k-opioid receptor (KOP) (PDB ID: 6B73), given the high sequence similarity with both SSTRs subtypes considered in this study. The quality of models was evaluated using the SWISS-MODEL workspace.

Three-dimensional structures of the ligands under investigation were built and correctly prepared taking advantage of the MOE suite.

GOLD docking tool was selected as a conformational search program and PLP as a scoring function. In total 20 docking runs were performed for each somatostatin receptor, searching in a sphere of 15 Å radius. Along with AE, the compound under investigation, docking simulations were conducted also for ligands L-779,976 and L-817,818, the references non-peptidic potent and selective agonists respectively of SSTR2 and SSTR5.

### RESULTS OF MOLECULAR MODELING

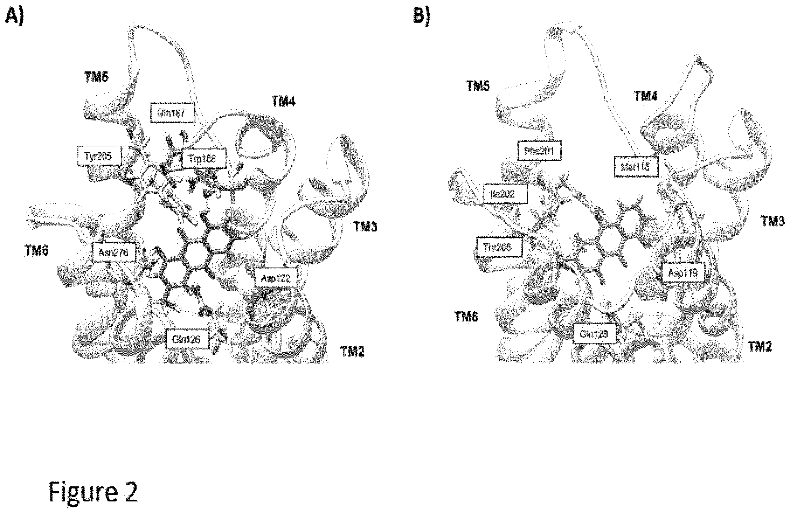

Since AE is a considerably smaller molecule than the endogenous agonist SST or the reference non-peptide compounds, the explorable space by the ligand within the orthosteric binding site is large enough to increase the variability of docking predicted poses. The most reasonable binding modes of AE, respectively within SSTR2 (Fig.2A) and SSTR5 binding sites (Fig.2B) are shown. AE interacts mainly with residues of the transmembrane segments TM3, TM5, TM6 and with the extracellular loop EL2, a portion of the binding site very similar to the one described for the reference agonist compound.

### Example 4

### CELL VIABILITY MODULATION BY SSTR-2 AND SSTR-5 ANTIBODIES AND SST14

In order to obtain a functional validation of the gene expression experiments and molecular dynamic prediction, cell viability modulation was studied using antibodies against SSTR2 and SSTR5 as well as the natural ligand SST14.

IMR5 cells were seeded as described above. After 24 h of incubation the medium was discarded and replaced by 100 µL of medium supplemented with SST14 (10µM), 1:500 SSTR2 Ab, SSTR5 Ab, or SSTR2 Ab and SSTR5 Ab together. Then IMR5 cells were tested with different concentration of AE, as mentioned before.

### RESULTS OF CELL VIABILITY MODULATION

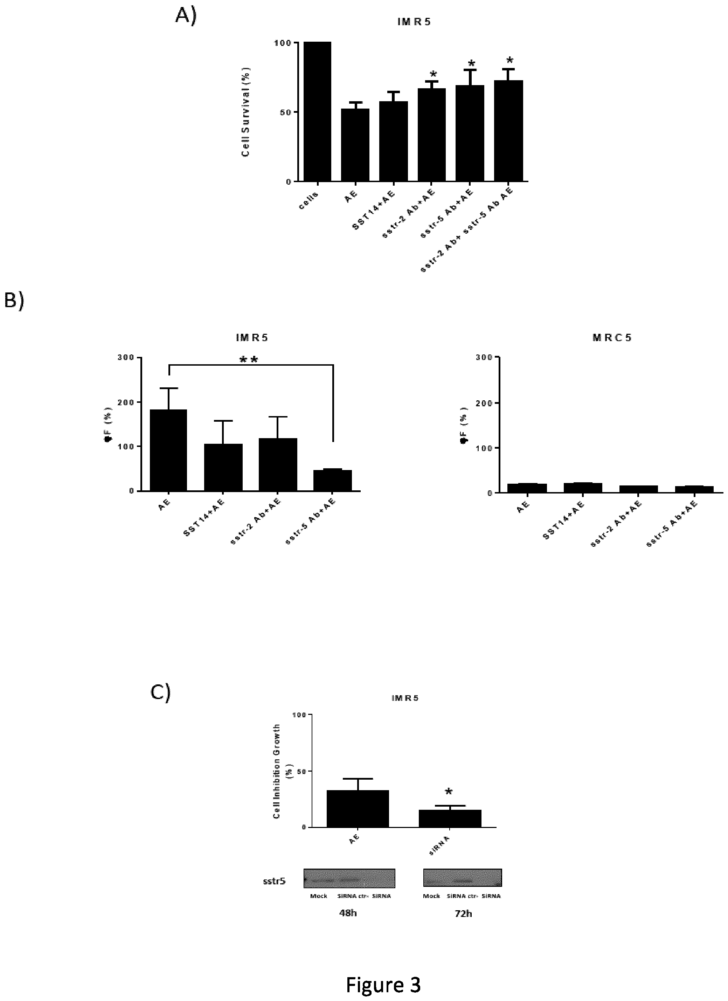

A significant increase in IMR5 cell survival was observed after 24 hours of co-exposition to AE and SSTR2 and SSTR5 antibodies. No increase in viability was observed in SST14 and AE co-treated cells. (Fig.3A).

### CONFOCAL MICROSCOPY ANALYSES

Being an autofluorescent molecule (λecc at 410 nm and λem at 610 nm) AE accumulation was investigated in sensitive and non-sensitive cells after treatment with antibodies and the somatostatin natural ligand, SST14.

IMR5 and MRC5 cells were seeded on microscope coverslips in 12-well plates and cultured with drugfree medium 24 h before treatment. Cells were treated with the compounds and observed in vivo. AE was added at the final concentration of 50µM. IMR5 and MRC5 cells were also pretreated with SST14 at 10 µM for 20 min and with SSTR5 and SSTR2 Abs (1:100) for 20 min.

AE fluorescence was collected during the incorporation and was evaluated as a ratio between the basal fluorescence of the cells and the maximum value of fluorescence reached in 3 min. Fluorescence was monitored with excitation at 488nm and emission at 500-550 nm. Confocal images were obtained using A1Rsi+Laser Scan Confocal microscope (Nikon Instruments Inc, Melville, USA) with a detection system and a 20x objective. Illumination intensity was 5% laser. Data are presented as change in fluorescence relative to the initial fluorescent value for each individual cell. Images were captured every 5 seconds. Analysis of fluorescence intensity was performed after image acquisition using NISElements Advanced research (Nikon Instruments Inc.).

### RESULTS OF CONFOCAL MICROSCOPY ANALYSES

Fluorescence was significantly down-regulated in IMR5 cells after incubation with the SSTR5 antibody; no such difference was present after SST14 and SSTR2 Ab pre-treatment; AE accumulation fluorescence was not evident in MRC5 cells treated or not treated with antibodies or SST14 (Fig. 3B).

### SIRNA-MEDIATED SSTR5 GENE KNOCKDOWN

IMR5 cells, as a representative cell line highly expressing SSTR5, were also transfected with sstr5 siRNA.

Gene-specific pooled siRNA trilencer targeting human sstr5 and a scrambled negative control duplex were purchased from Origene (OriGene Technologies, Rockville, MD, USA). IMR5 cells were transfected with 20 nM aliquots of human sstr5 siRNA and control siRNA by using Lipofectamine RNAiMAX (Invitrogen, Thermo Fisher Scientific, Waltham, MA, USA) following the manufacturer's instructions. Twentyfour h before transfection 30,000 cells were plated in 500 µl of growth medium without antibiotics for 24 h. Then the cells were transfected with RNAi duplex-Lipofectamine^{™} RNAiMAX complexes, final RNA concentration of 20 nM, for 20 minutes at room temperature.

Cells were incubated at 37°C in a CO₂ incubator until assayed for gene knockdown. Gene silencing was stable for 72 h. After 48h cells were treated with AE (50 µM). 24 hours after AE treatment cells were harvested and prepared for quantitative proliferation assay, as previously described. Non-targeting siRNA transfected cells were used as a negative control.

### RESULTS OF GENE KNOCKDOWN

After gene silencing cells were significantly less sensitive to AE growth inhibition (Fig.3C).

### Example 5

### IMMUNOSTAINING

To further investigate the possible role of SSTR in AE treatment of neuroblastoma in vivo, we evaluated SSTR2 and SSTR5 immunoreactivity in neuroblastoma's surgical specimens.

Paraffin embedded surgical neuroblastoma specimens (n=9) were retrieved from the archives of the Surgical Pathology Unit of the University Hospital of Padua. Immunostaining was automatically performed using the Bond Polymer Refine Detection kit (Leica Biosystems, Newcastle Upon Tyne, UK) in the BOND-MAX system (Leica Biosystems) on 3-4 µm-thick sections with the primary antibodies for SSTR2 (anti-somatostatin receptor 2 antibody [UMB1]; Abcam, Cambridge, UK) and SSTR5 (antisomatostatin receptor 5 antibody [UMB4]; Abcam, Cambridge, UK).

The staining intensity thresholds were set as follows: strong, (3+); moderate, (2+); weak, (1 +). The total number of cells for each staining intensity was calculated and a modified H-score was calculated for each tumor with the following formula: (1×[% cells 1+]+2x[% cells 2+]+3×[% cells 3+])/100. The scores lay on a continuous scale from 0 to 3 (20).

### RESULTS OF IMMUNOSTAINING ANALYSES

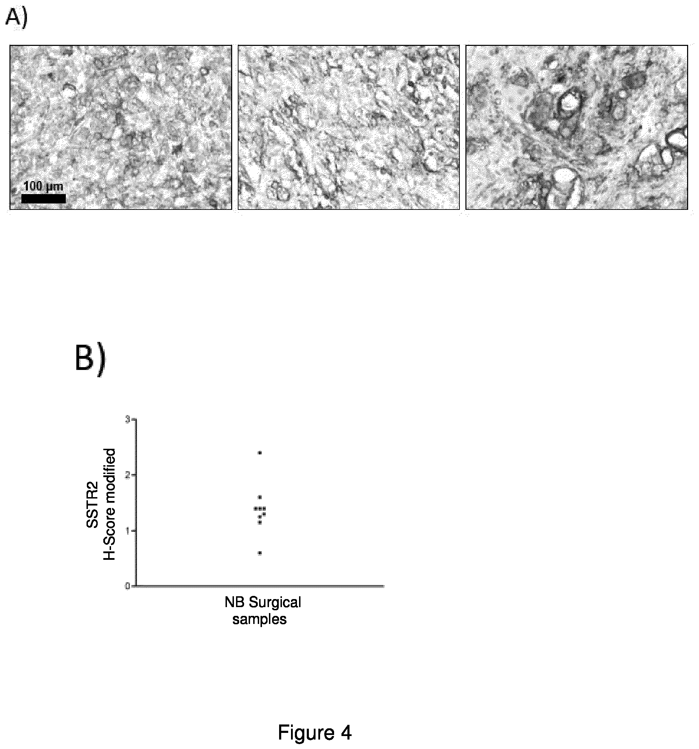

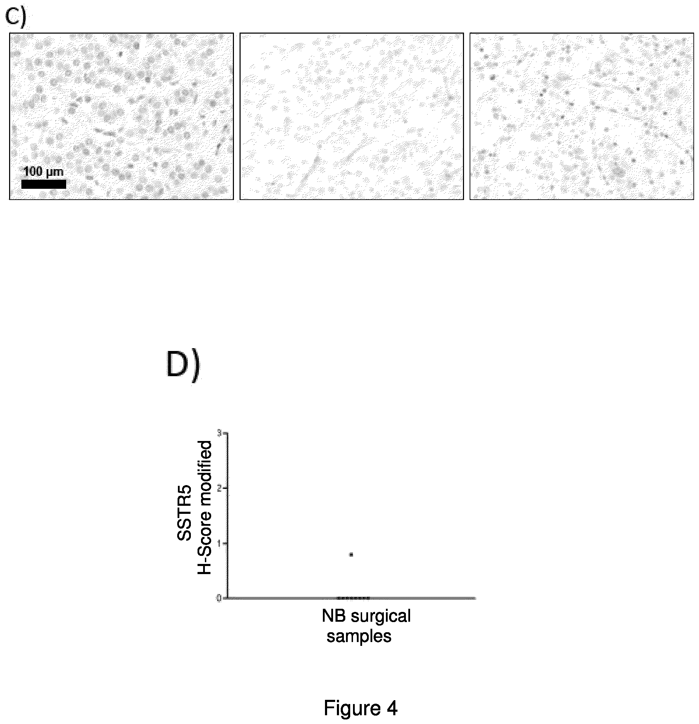

While SSTR5 immunoreactivity was barely detectable in 1 out of the nine tumour specimens evaluated, a moderate to strong immunoreactivity for SSTR2 was detectable in all neuroblastoma sections examined (Fig 4).

### Example 6

### EVALUATION OF AE ACTIVITY ON EX VIVO ACTIVATED MURINE MACROPHAGES

The experiments were conducted on murine peritoneal macrophages.

The macrophages were collected by peritoneal washing 10 days after the intraperitoneal inoculation of sterile thioglycolate. The cells were centrifuged, counted and seeded in well plates. After 48 hours the medium was discarded and the cells incubated with AE (0.1-50 µM) or with vehicle only. After 60 minutes LPS (100 ng / ml) or vehicle only was added to the medium. The cells were incubated for 24 hours, at the end of which the culture medium was collected for quantification by means of the ELISA assay of the cytokine IL-1β. The cells were incubated for a further two hours with a fluorescent probe capable of irreversibly binding the active Caspase-1 (key enzyme of the inflammosome). At the end of incubation the cells were harvested, labeled with an anti-CD11b fluorescent antibody (specific for macrophages) and then analyzed at the FACS to evaluate the percentage of macrophages with double positivity (CD11b + caspase1 activated).

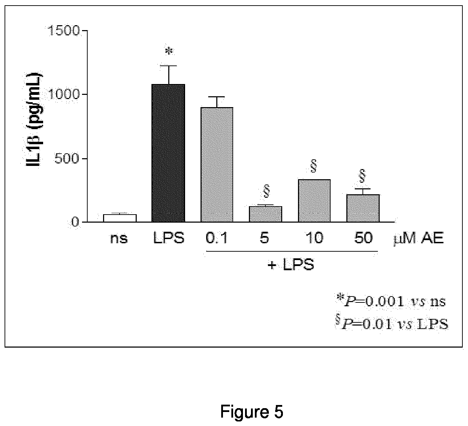

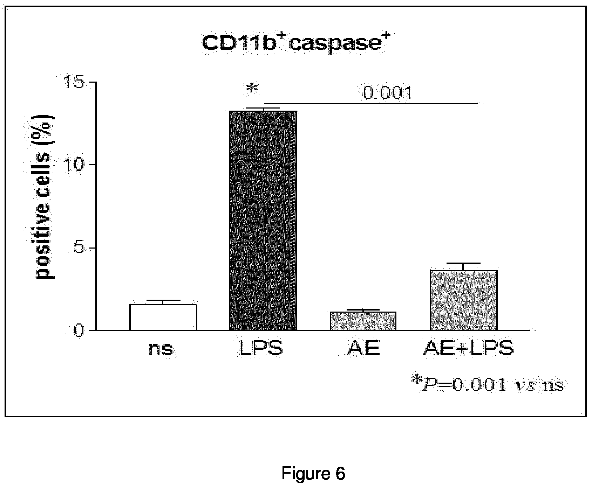

Figures 5 and 6 show the results of two different experiments conducted with quadruplicate (ELISA) or duplicate (caspase1) determinations.

### RESULTS OF AE ACTIVITY ON EX VIVO ACTIVATED MURINE MACROPHAGES

From the preliminary tests performed it is evident that in the presence of AE starting from concentrations of 5 µM, the secretion of IL-1 β induced by LPS in macrophages is almost completely abolished. In addition, the 5 µM) dose of AE almost completely inhibits the activation of Caspase1, a key enzyme in the production of IL-1β in murine macrophages.

Our preliminary data indicate that AE binds somatostatin receptors 2 and 5 which allow its internalization. Receptors 2 and 3 for somatostatin are over-expressed in activated macrophages.

### STATISTICAL ANALYSES

Statistical analyses were performed with Student's unpaired t-test with *P<0.05 and **P< 0,01.

From the above description and the above-noted examples, the advantage attained by the use of 1,8-dihydroxy-3-[hydroxymethyl]-anthraquinone compounds described and obtained according to the present invention are apparent.

## Claims

1. 1,8-dihydroxy-3-[hydroxymethyl]-anthraquinone or derivatives thereof, for use in the treatment of diseases in which somatostatin receptor 2 (SSTR2) and/or somatostatin receptor 5 (SSTR5) are overexpressed, wherein said derivatives are 3'- esters of 1,8-dihydroxy-3-[hydroxymethyl]-anthraquinone with an amino acid chosen from the group consisting of lysine, alanine, tryptophan, isoleucine, tyrosine and GABA, wherein said diseases are tumors chosen from the group consisting of neuroblastoma, neuroendocrine tumors or primitive neuroectodermal tumors (pNETS).

2. The compound for use according to claim 1, wherein said 1,8-dihydroxy-3-[hydroxymethyl]-anthraquinone or derivatives thereof are in the form of pharmaceutical formulations for drug administration by the parenteral, oral, and local administration.

3. The compound for use according to claims 1 to 2 wherein said 1,8-dihydroxy-3-[hydroxymethyl]-anthraquinone or derivatives thereof are in the range from 5 to 500 mg for unit administration.

## Patentansprüche

1. 1,8-Dihydroxy-3-[hydroxymethyl]-anthrachinon oder Derivate davon zur Verwendung bei der Behandlung von Krankheiten, bei denen der Somatostatinrezeptor 2 (SSTR2) und/oder der Somatostatinrezeptor 5 (SSTR5) überexprimiert sind, wobei die Derivate 3'-Ester von 1,8-Dihydroxy-3-[hydroxymethyl]-anthrachinon mit einer Aminosäure sind, die aus der Gruppe ausgewählt ist, die aus Lysin, Alanin, Tryptophan, Isoleucin, Tyrosin und GABA besteht, wobei die Krankheiten Tumore sind, die aus der Gruppe ausgewählt sind, die aus Neuroblastom, neuroendokrinen Tumoren oder primitiven neuroektodermalen Tumoren (pNETS) besteht.

2. Verbindung zur Verwendung nach Anspruch 1, wobei das 1,8-Dihydroxy-3-[hydroxymethyl]-anthrachinon oder Derivate davon in Form von pharmazeutischen Formulierungen zur Verabreichung von Medikamenten durch parenterale, orale und lokale Verabreichung vorliegen.

3. Verbindung zur Verwendung nach den Ansprüchen 1 bis 2, wobei das 1,8-Dihydroxy-3-[hydroxymethyl]-anthrachinon oder Derivate davon im Bereich von 5 bis 500 mg für Einheitsverabreichung liegt.

## Revendications

1. 1,8-dihydroxy-3-[hydroxyméthyl]-anthraquinone ou ses dérivés, pour une utilisation dans le traitement de maladies dans lesquelles le récepteur 2 de la somatostatine (SSTR2) et/ou le récepteur 5 de la somatostatine (SSTR5) sont surexprimés, dans lesquelles lesdits dérivés sont des esters 3' de 1,8-dihydroxy-3-[hydroxyméthyl]-anthraquinone avec un acide aminé choisi dans le groupe constitué par la lysine, l'alanine, le tryptophane, l'isoleucine, la tyrosine et le GABA, dans lequel lesdites maladies sont des tumeurs choisies dans le groupe constitué par les neuroblastomes, les tumeurs neuroendocrines ou les tumeurs neuroectodermiques primitives (pNETS).

2. Composé pour une utilisation selon la revendication 1, dans lequel ladite 1,8-dihydroxy-3-[hydroxyméthyl]-anthraquinone ou ses dérivés se présentent sous la forme de formulations pharmaceutiques pour l'administration de médicaments par voie parentérale, orale et locale.

3. Composé pour une utilisation selon les revendications 1 à 2, dans lequel ladite 1,8-dihydroxy-3-[hydroxyméthyl]-anthraquinone ou ses dérivés sont compris entre 5 et 500 mg pour une administration unitaire.
